# EUROPEAN PATENT APPLICATION

(11) **EP 3 498 212 A1**
(43) Date of publication of application: **19.06.2019**
(21) Application number: 18211806.7
(22) Date of filing: 12.12.2018
(51) Int. Cl.: A61B 34/20, A61B 90/00

(54) **A METHOD FOR PATIENT REGISTRATION, CALIBRATION, AND REAL-TIME AUGMENTED REALITY IMAGE DISPLAY DURING SURGERY**

(30) Priority: 12.12.2017 EP 17206557
(71) Applicant: Holo Surgical Inc., Chicago, IL 60609 (US)
(72) Inventor: Siemionow, Kris B., Chicago, Illinois 60610 (US); Luciano, Cristian J., Evergreen Park, Illinois 60805 (US)
(74) Representative: Kancelaria Eupatent.pl Sp. z.o.o

(57) **Abstract**

A method for registering patient anatomical data (163) in a surgical navigation system, the method comprising the following steps: by means of a medical scanner (180), scanning (502) a volume comprising a patient anatomy of interest and a registration grid (181) placed at a first position (105A) over the surgical field, to obtain patient anatomical data (163), wherein the registration grid (181) comprises a plurality of fiducial markers (181B); by means of a fiducial marker tracker (125), capturing (504) the 3D positions and orientations of a pre-attached anatomy array (123) attached to the patient at a second position (105B) around the surgical field and of the registration grid (181) placed over the surgical field (105A), wherein the anatomy array (123) comprises a plurality of fiducial markers (123B); and registering (506) the patient anatomical data (163) with respect to the 3D position and orientation of the anatomy array (123) determined as a function of the relative positions and orientations of the registration grid (181) and the anatomy array (123).

## Description

### TECHNICAL FIELD

The present disclosure relates to surgical navigation systems, in particular to a system and method for operative planning, image acquisition, patient registration, calibration, and execution of a medical procedure using an augmented reality image display.

### BACKGROUND

Some of typical functions of a computer-assisted surgery (CAS) system with navigation include presurgical planning of a procedure and presenting preoperative diagnostic information and images in useful formats. The CAS system presents status information about a procedure as it takes place in real time, displaying the preoperative plan along with intraoperative data. The CAS system may be used for procedures in traditional operating rooms, interventional radiology suites, mobile operating rooms or outpatient clinics. The procedure may be any medical procedure, whether surgical or non-surgical.

Surgical navigation systems are used to display the position and orientation of surgical instruments and medical implants with respect to presurgical or intraoperative medical imagery datasets of a patient. These images include pre and intraoperative images, such as two-dimensional (2D) fluoroscopic images and three-dimensional (3D) magnetic resonance imaging (MRI) or computed tomography (CT).

Navigation systems locate markers attached or fixed to an object, such as surgical instruments and patient. Most commonly these tracking systems are optical and electro-magnetic. Optical tracking systems have one or more stationary cameras that observes passive reflective markers or active infrared LEDs attached to the tracked instruments or the patient. Eye-tracking solutions are specialized optical tracking systems that measure gaze and eye motion relative to a user's head. Electro-magnetic systems have a stationary field generator that emits an electromagnetic field that is sensed by coils integrated into tracked medical tools and surgical instruments.

Incorporating image segmentation processes that automatically identify various bone landmarks, based on their density, can increase planning accuracy. One such bone landmark is the spinal pedicle, which is made up of dense cortical bone making its identification utilizing image segmentation easier. The pedicle is used as an anchor point for various types of medical implants. Achieving proper implant placement in the pedicle is heavily dependent on the trajectory selected for implant placement. Ideal trajectory is identified by surgeon based on review of advanced imaging (e.g., CT or MRI), goals of the surgical procedure, bone density, presence or absence of deformity, anomaly, prior surgery, and other factors. The surgeon then selects the appropriate trajectory for each spinal level. Proper trajectory generally involves placing an appropriately sized implant in the center of a pedicle. Ideal trajectories are also critical for placement of inter-vertebral biomechanical devices.

Another example is placement of electrodes in the thalamus for the treatment of functional disorders, such as Parkinson's. The most important determinant of success in patients undergoing deep brain stimulation surgery is the optimal placement of the electrode. Proper trajectory is defined based on preoperative imaging (such as MRI or CT) and allows for proper electrode positioning.

Another example is minimally invasive replacement of prosthetic/biologic mitral valve in for the treatment of mitral valve disorders, such as mitral valve stenosis or regurgitation. The most important determinant of success in patients undergoing minimally invasive mitral valve surgery is the optimal placement of the three dimensional valve.

The fundamental limitation of surgical navigation systems is that they provide restricted means of communicating to the surgeon. Currently-available navigation systems present some drawbacks.

Typically, one or several computer monitors are placed at some distance away from the surgical field. They require the surgeon to focus the visual attention away from the surgical field to see the monitors across the operating room. This results in a disruption of surgical workflow. Moreover, the monitors of current navigation systems are limited to displaying multiple slices through three-dimensional diagnostic image datasets, which are difficult to interpret for complex 3D anatomy.

The fact that the screen of the surgical navigation system is located away from the region of interest (ROI) of the surgical field requires the surgeon to continuously look back and forth between the screen and the ROI. This task is not intuitive and results in a disruption to surgical workflow and decreases planning accuracy.

For example, a system of such type is disclosed in a US patent US9532848, which discloses a system for assisting a user manipulating an object during a surgery, the system comprising a tracking device for tracking the object and for generating tracking data for the object and a sterilized displaying device located in a volume within the sterile field defined as being above a plane of and delimited by an operating table and below the shoulders of an operator standing next to a patient lying on the operating table, the displaying device being supported directly by the operating table. Even though the displaying device is positioned very close to the patient being operated, the surgeon still needs to look back and forth between the screen and the ROI.

When defining and later executing an operative plan, the surgeon interacts with the navigation system via a keyboard and mouse, touchscreen, voice commands, control pendant, foot pedals, haptic devices, and tracked surgical instruments. Based on the complexity of the 3D anatomy, it can be difficult to simultaneously position and orient the instrument in the 3D surgical field only based on the information displayed on the monitors of the navigation system. Similarly, when aligning a tracked instrument with an operative plan, it is difficult to control the 3D position and orientation of the instrument with respect to the patient anatomy. This can result in an unacceptable degree of error in the preoperative plan that will translate to poor surgical outcome.

There is therefore a need to improve the surgical navigation systems to eliminate the above-mentioned drawbacks.

A GB patent application GB2536650 discloses a system and method for a near-eye display comprising at least one each of an optical combiner with a selective shutter layer at one side, a scene capturing device, a micro projector to project the captured video and a processor to determine at least one region of interest selected by a user and controlling the shutter layer to reduce the amount of light transferable via a portion of the optical combiner corresponding to the determined region.

A US patent application US20170042631 discloses an intra-operative medical image viewing system comprising a display positionable between a surgeon and the patient during surgery, said display being configured to exhibit images to the surgeon overlaid on the patient; an image control unit configured to retrieve at least one image file from an image source and control said display to exhibit and modify at least a portion of the at least one image; and a plurality of peripheral devices, each configured to receive an image control input from the surgeon and in response to generate an image control signal in a respective user-interface modality. In one embodiment, the display is a wearable device, such as three-dimensional glasses. In another embodiment, the display is a see-through screen that is mounted on a non-wearable frame that is fixed, as seen in the embodiment figure, closer to the patient than to the surgeon's head. The display is capable of locating a medical image between the eyes of the surgeon and the patient. The system can fuse together multiple images, such as pre-operative and/or intra-operative images to conform to the surgeon's visual perspective.

The above-mentioned solutions partially eliminate the drawback of typical prior art surgical navigation systems wherein the computer monitors are placed at some distance away from the operative field and require the surgeon to focus the visual attention away from the surgical field to see the monitors across the operating room.

However, one drawback related to head-worn apparatus, such as three-dimensional display glasses disclosed in the above-mentioned publications, is that they must provide high-resolution display to generate a signal of satisfactory quality, in particular for a large field of view. Moreover, the head-worn displays have a limited field of view. Moreover, they need very precise positioning control, as the slightest changes of surgeon's head require repositioning of the displayed image - it's quite hard to precisely and consistently keep the head-mounted display at exactly the same position and orientation with respect to the surgeon's eyes, perfect virtual/real collocation of patient anatomy is difficult to achieve. Augmented reality head-mounted devices are designed to display virtual images located at roughly the same height as the user's head, and at a certain distance from the user's body, which is not appropriate for surgery. This makes almost impossible to achieve virtual/real anatomical superposition at a location that is reachable by the surgeon's hands. Also, the significant weight of the head-worn apparatus or head mounted display (HMD) can produce tiredness on the surgeon after a long period of usage. The surgeon has limited range of movements, it makes it harder to maneuver the head during the surgery and talk to other physicians. Moreover, use of head mounted displays involves vergence-accommodation conflict, that causes fatigue and discomfort of the user.

These drawbacks can be avoided partially by providing a fixed non-wearable see-through screen, that may have a larger display area and therefore high-resolution image is easier to be displayed therein. However, the frame as disclosed in US20170042631, being located closer to the patient than to the surgeon's head, may obstruct the freedom of instrument and hand movement during the operating procedure.

Workflow efficiency in the operating room is critical for proper execution of surgical procedures. Disruptions to workflow or system inefficiency prolong procedure times, distract the surgeon and the operating room staff, and result in higher error rates. This results in decrease utilization of inefficient surgical systems. Augmented reality display CAS systems (whether head mounted or not) have the potential to improve workflow by allowing the surgeon to visualize key anatomical structures without distraction or taking the surgeon's eyes off the surgical field.

The augmented reality systems allow overlaying a virtual image over a real-world image, such that these images are correctly collocated, depending on the viewpoint of the surgeon. In order to do so, it is essential to track the position of the surgeon's head and direction of view with respect to the real anatomy. This, in turn, requires to perform a preoperative scan of the real anatomy and to register the scan with respect to the same coordinate system in which the surgeon's head is tracked. Performing and registering a pre-operative scan of patient anatomy is not a trivial task.

There is, therefore, a need to further improve on the method of performing augmented reality surgical navigation and to eliminate at least some of the above-mentioned drawbacks.

### SUMMARY

There is disclosed a method for registering patient anatomical data in a surgical navigation system, the method comprising the following steps: placing a registration grid over the patient at a first position, wherein the registration grid comprises a plurality of fiducial markers; by means of a medical scanner, scanning both a patient anatomy of interest and the registration grid to obtain patient anatomical data; attaching an anatomy array comprising a plurality of fiducial markers to the patient at a second position; by means of a fiducial marker tracker, capturing the 3D positions and orientations of the anatomy array and the registration grid; and registering the patient anatomical data with respect to the 3D position and orientation of the anatomy array determined as a function of the relative positions and orientations of the registration grid and the anatomy array.

The anatomy array may be attached to the patient internal anatomy around the surgical field

The fiducial marker tracker may use an optical, electromagnetic or acoustic technology for capturing the 3D position and orientation.

The method may further comprise: using the tracker for real-time tracking of the anatomy array; generating, by a surgical navigation image generator, a surgical navigation image comprising the patient anatomical data adjusted with respect to the 3D position and orientation of the anatomy array; showing the surgical navigation image by means of a 3D display system such that an augmented reality image, collocated with the surgical field, is visible to a viewer looking towards the surgical field.

The method may further comprise: by means of the tracker, real-time tracking of at least one of: a surgeon's head, a 3D display and surgical instruments to provide current 3D position and orientation data; and adjusting the surgical navigation image with respect to the tracked current 3D position and orientation data.

The method may further comprise: by means of the tracker, real-time tracking of a robotic arm marker array; and generating the surgical navigation image further comprising the robotic arm virtual image adjusted with respect to the tracked position and orientation of the robotic arm marker array.

The method may further comprise identifying the fiducial markers of the registration grid in the volumetric data provided by the medical scanner using a convolutional neural network.

The intended use of this invention is both presurgical planning of ideal surgical instrument trajectory and placement, and intraoperative surgical guidance, with the objective of helping to improve surgical outcomes.

### BRIEF DESCRIPTION OF DRAWINGS

The surgical navigation system and method are presented herein by means of nonlimiting example embodiments shown in a drawing, wherein:
Fig. 1 shows a layout of a surgical room employing a surgical navigation system;
Fig. 2 shows components of the surgical navigation system;
Figs. 3A, 3B show examples of an augmented reality display;
Fig. 4 shows an overview of the operating room during the registration procedure;
Fig. 5 shows an overview of a method for patient anatomy registration;
Figs. 6A-6D show details related to the patient anatomy registration.

### DETAILED DESCRIPTION

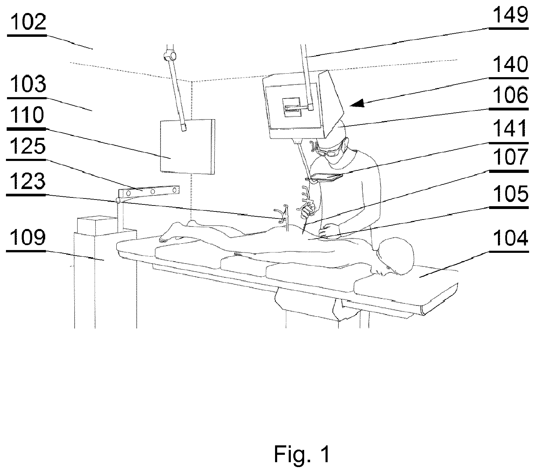

Figs. 1, 2 and 3 show an example of a surgical navigation system employing augmented reality display, for which the method for patient registration as described later herein is applicable. This is only an example and the method for patient registration can be used with other systems as well.

The example of the surgical navigation system as presented herein in Fig. 1 comprises a 3D display system 140 to be implemented directly on real surgical applications in a surgical room. The 3D display system 140 as shown in the example embodiment comprises a 3D display 142 for emitting a surgical navigation image 142A towards a see-through mirror 141 that is partially transparent and partially reflective, such that an augmented reality image 141A collocated with the patient anatomy in the surgical field 108 underneath the see-through mirror 141 is visible to a viewer looking from above the see-through mirror 141 towards the surgical field 108.

A patient 105 lies on the operating table 104 while being operated by a surgeon 106 with the use of various surgical instruments 107. The surgical navigation system as described in details below can have its components, in particular the 3D display system 140, mounted to a ceiling 102, or alternatively to the floor 101 or a side wall 103 of the operating room. Furthermore, the components, in particular the 3D display system 140, can be mounted to an adjustable and/or movable floor-supported structure (such as a tripod). Components other than the 3D display system 140, such as the surgical image generator 131, can be implemented in a dedicated computing device 109, such as a stand-alone PC computer, which may have its own input controllers and display(s) 110.

In addition, the system may comprise a robotic arm 191 for handling some of the surgical tools. The robotic arm 191 may have two closed loop control systems: its own position system and one used with the optical tracker as presented herein. Both systems of control may work together to ensure that the robotic arm is on the right position. The robotic arm's position system may comprise encoders placed at each joint to determine the angle or position of each element of the arm. The second system may comprise a robotic arm marker array 126 attached to the robotic arm to be tracked by the tracker 125, as described below. Any kind of surgical robotic system can be used, preferably one that follows FDA standards.

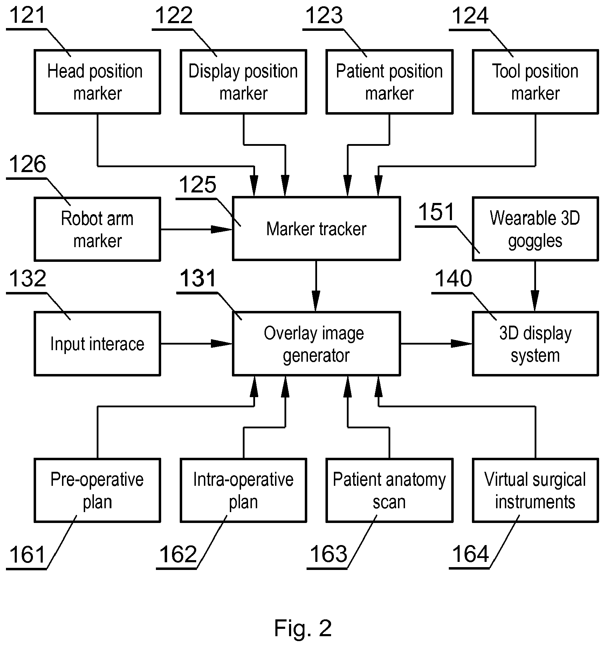

Fig. 2 shows a functional schematic presenting connections between the components of the surgical navigation system.

The surgical navigation system comprises a tracking system for tracking in real time the 3D (i. e. in three dimensions) position and orientation of various entities to provide current position and orientation data. For example, the system may comprise a plurality of arranged fiducial markers, which are trackable by a fiducial marker tracker 125. Any known type of tracking system can be used, for example in case of a marker tracking system, 4-point marker arrays are tracked by a three-camera sensor to provide movement along six degrees of freedom. A head position marker array 121 can be attached to the surgeon's head for tracking of the position and orientation of the surgeon and the direction of gaze of the surgeon - for example, the head position marker array 121 can be integrated with the wearable 3D glasses 151 or can be attached to a strip worn over surgeon's head.

Alternatively, the tracker may use optical, electromagnetic, acoustic, or any other technology for capturing the 3D position and orientation of markers.

A display marker array 122 can be attached to the see-through mirror 141 of the 3D display system 140 for tracking its position and orientation, as the see-through mirror 141 is movable and can be placed according to the current needs of the operative setup.

A patient anatomy marker array 123, also called herein an anatomy array 123 or a tracking array, can be attached at a particular position and orientation of the anatomy of the patient.

A surgical instrument marker array 124 can be attached to the instrument whose position and orientation shall be tracked.

A robotic arm marker array 126 can be attached to at least one robotic arm 191 to track its position.

Preferably, the markers in at least one of the marker arrays 121-124 are not coplanar, which helps to improve the accuracy of the tracking system.

Therefore, the tracking system comprises means for real-time tracking of the position and orientation of: a surgeon's head 106, a 3D display 142, a patient anatomy 105, and surgical instruments 107. Preferably, all of these elements are tracked by a fiducial marker tracker 125.

A surgical navigation image generator 131 is configured to generate an image to be viewed via the see-through mirror 141 of the 3D display system. It generates a surgical navigation image 142A comprising data of at least one of: the pre-operative plan 161 (which are generated and stored in a database before the operation), data of the intra-operative plan 162 (which can be generated live during the operation), data of the patient anatomy scan 163 (which can be generated before the operation or live during the operation) and virtual images 164 of surgical instruments used during the operation (which are stored as 3D models in a database), as well as virtual image 166 of the robotic arm 191.

The surgical navigation image generator 131, as well as other components of the system, can be controlled by a user (i.e. a surgeon or support staff) by one or more user interfaces 132, such as foot-operable pedals (which are convenient to be operated by the surgeon), a keyboard, a mouse, a joystick, a button, a switch, an audio interface (such as a microphone), a gesture interface, a gaze detecting interface etc. The input interface(s) are for inputting instructions and/or commands.

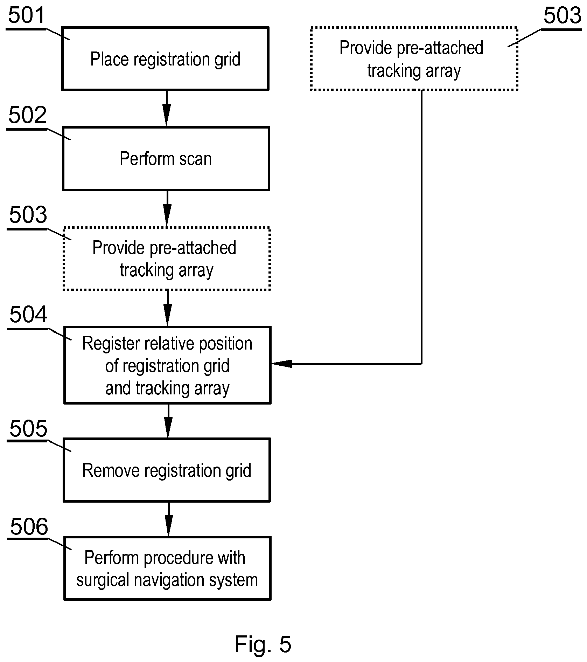

The surgical navigation image generator 131 is configured to control the steps of the method described with reference to Fig. 5 and calculate necessary data to perform the method.

All system components are controlled by one or more computer which is controlled by an operating system and one or more software applications. The computer may be equipped with a suitable memory which may store computer program or programs executed by the computer in order to execute steps of the methods utilized in the system. Computer programs are preferably stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein. The computer(s) can be placed within the operating room or outside the operating room. Communication between the computers and the components of the system may be performed by wire or wirelessly, according to known communication means.

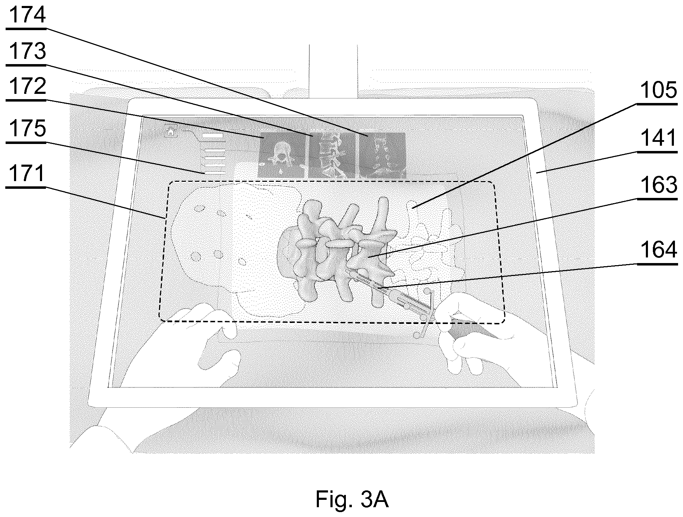

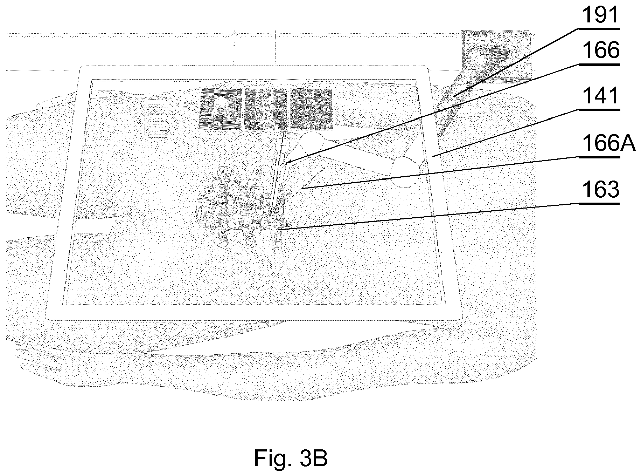

The aim of the system is to generate, via the 3D display system 140, an augmented reality image such as shown in Fig. 3A or 3B. When the surgeon looks via the 3D display system 140, the surgeon sees the augmented reality image 141A which comprises:
- the real world image: the patient anatomy, surgeon's hands and the instrument currently in use (which may be partially inserted into the patient's body and hidden under the skin);
- and a computer-generated surgical navigation image 142A comprising at least one of: the pre-operative plan 161, data of the intra-operative plan 162, data of the patient anatomy scan 163 (supplemented by different orthogonal planes of the patient anatomical data 163: coronal 174, sagittal 173, axial 172), virtual images 164 of surgical instruments used during the operation, virtual image 166 of the robotic arm, a menu 175 for controlling the system operation.

If the 3D display 142 is stereoscopic, the surgeon shall use a pair of 3D glasses 151 to view the augmented reality image 141A. However, if the 3D display 142 is autostereoscopic, it may be not necessary for the surgeon to use the 3D glasses 151 to view the augmented reality image 141A.

The surgical navigation image 142A is generated by the image generator 131 in accordance with the tracking data provided by the fiducial marker tracker 125, in order to superimpose the anatomy images and the instrument images exactly over the real objects, in accordance with the position and orientation of the surgeon's head. The markers are tracked in real time and the image is generated in real time. Therefore, the surgical navigation image generator 131 provides graphics rendering of the virtual objects (patient anatomy, surgical plan and instruments) collocated to the real objects according to the perspective of the surgeon's perspective.

The 3D display system described above makes use of a 3D display 142 with a see-through mirror 141, which is particularly effective to provide the surgical navigation data. However, other 3D display systems can be used as well to show the augmented image, such as 3D head-mounted displays.

The virtual image of the patient anatomy 163 is generated based on data representing a three-dimensional segmented model comprising at least two sections representing parts of the anatomy. The anatomy can be for example a bone structure, such as a spine, skull, pelvis, long bones, shoulder joint, hip joint, knee joint etc. This description presents examples related particularly to a spine, but a skilled person will realize how to adapt the embodiments to be applicable to the other bony structures or other anatomy parts as well.

The model is generated based on a pre-operative scan of the patient.

The following description will present a method for registering a pre-operative or intra-operative scan of the patient.

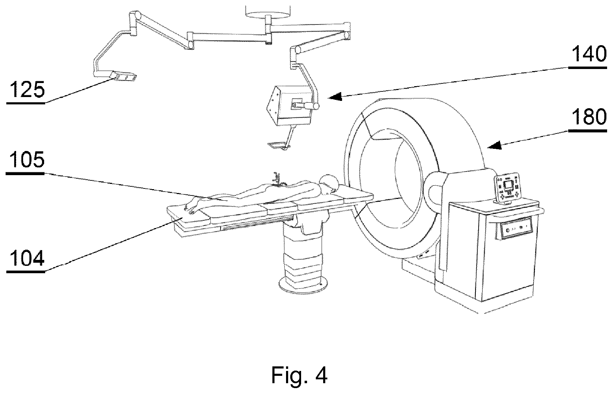

Fig. 4 shows an overview of the operating room, with the elements shown that are similar to that shown in Fig. 1. In addition, a medical intraoperative image scanner (IIS) 180 is present, to perform patient anatomy scanning to obtain the patient anatomical data 163. The presented example of the IIS 180 is a computer tomography (CT) scanner, but other types of scanners can be used as well.

Fig. 5 shows steps of a method for patient anatomy registration and other supportive actions.

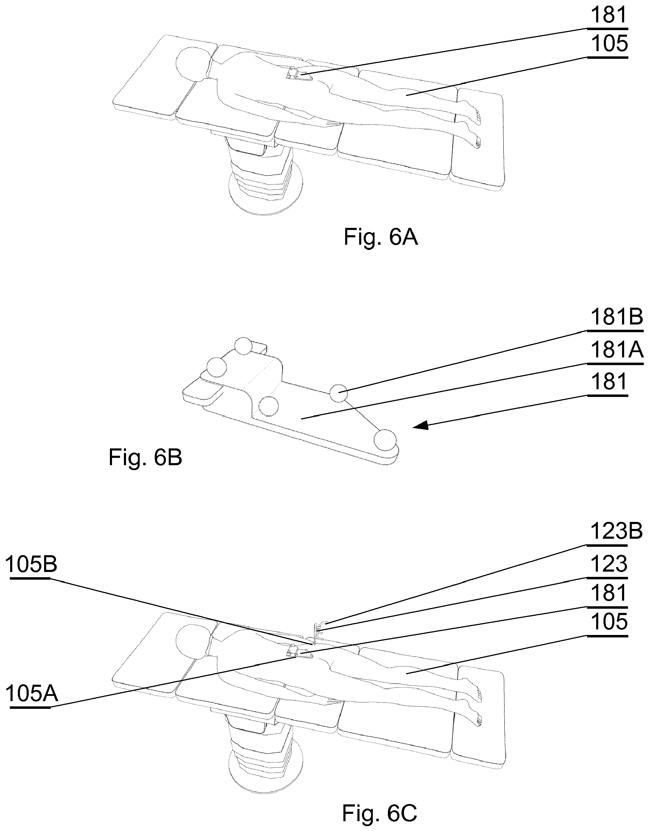

In step 501, a registration grid 181 is placed on the patient at a first position over the surgical field 105A, as shown in Fig. 6A.

In step 502, a volume comprising the patient anatomy of interest and the registration grid is scanned with the IIS 180.

The registration grid 181 is a device that has a base 181A and an array of fiducial markers 181B that are registrable both by the IIS 180 and the fiducial marker tracer 125. For highly accurate registration results, the grid 181 may comprise five markers 181B, forming three groups of three markers each (some markers may belong to more than one group), each group arranged on a different plane. The registration grid 181 can be attached to the patient for example by an adhesive, such that it stays in the position during the scan. Registration grids with other amount and arrangement of markers can be used as well, depending on the needs.

The fiducial markers of the registration grid 181 in the volumetric data provided by the medical scanner can be identified using a convolutional neural network (CNN).

Therefore, the scan of the patient anatomy performed in step 502 comprises data of the patent anatomy and of the registration grid, in particular the markers 181B.

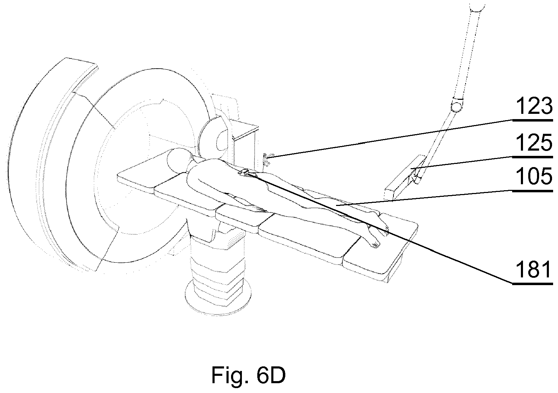

In step 503, the anatomy array 123 is provided that is pre-attached to the patient, at a second position 105B, as shown in Fig. 6C. Attachment of the anatomy array 123 to the patient can be done in a surgical or a non-surgical procedure. For example, the anatomy array can be inserted into the iliac crest or a bony anchor point. The anatomy array can be also attached by means of a dedicated holder to the body of the patient that does not require invasion inside the human body. The anatomy array 123 can be attached to the patient in step 501, along with the grid 181 or even before step 501. In step 504, the fiducial marker tracker 125 is used to capture and record the 3D position and orientation of both the anatomy array 123 and the registration grid 181, as shown in Fig. 6D. During this section of the process, the relative position and orientation of the anatomy array 123 and the registration grid 181 is determined. The relative position and orientation works as the reference to keep track of the position and orientation of the anatomy of the patient when the registration grid 181 is removed.

Steps 501, 503 related to arrangement of the registration grid 181, anatomy array 123, and of the fiducial marker tracker 125 with respect to the patient body can be performed by different personnel than the steps of scanning 502 and capturing 504 of images. The step 503 can be performed prior step 502.

Next, in step 505, the registration grid 181 can be removed from the patient. Even though the registration grid is removed, the patient's anatomy is still properly tracked because the anatomy array 123 keeps that relative reference to display the anatomy in place.

In step 506, the patient anatomical data 163 is registered with respect to the 3D position and orientation of the anatomy array 123 as a function of the relative position and orientation of the registration grid 181, anatomy array 123, and the fiducial marker tracker 125, in particular as the function of their fiducial markers 181B, 123B. The 3D display system may be then activated to present an augmented reality image, such as shown in Fig. 3A or 3B. The patient anatomy virtual image can be then collocated with the real physical anatomy of the patient. Therefore, the augmented reality image comprises the patient anatomical data 163 (as well as other virtual images, such as virtual instrument images 164) registered with respect to the position of the anatomy array 123, which is displayed considering the 3D position and orientation of the structure system 140 and the surgeon's head 106.

The surgical procedure can be performed now with the use of the surgical navigation system, wherein the patient anatomical data 163 is precisely aligned with the position and orientation of the anatomy array 123, which are real-time tracked by the fiducial marker tracker 125 during the surgical procedure.

Moreover, if some of the surgical tools can be handled by the robotic arm 191, their position and orientation are tracked via the robotic arm marker array 126. The augmented reality image may further comprise a virtual image 166 of the robotic arm collocated with the real physical anatomy of the patient, as shown in Fig. 3B. Furthermore, the augmented reality image may comprise a guidance image 166A that indicates, according to the preoperative plan data, the suggested position and orientation of the robotic arm 191.

The virtual image 166 of the robotic arm may be configurable such that it can be selectively displayed or hidden, in full or in part (for example, some parts of the robotic arm, such as its forearm, can be hidden and some other, such as the surgical tool holder, can be visible). Moreover, the opacity of the robotic arm virtual image 166 can be selectively changed, such that it does not obstruct the patient anatomy.

The advantage of the presented method is that the patient anatomical data 163 is precisely scanned by the IIS 180 along with the registration grid 181, which can be positioned at the first position 105A in a very close vicinity of the area of interest to be scanned, therefore an accurate scan of the anatomy and the grid can be performed. After the scan is complete, the position and orientation of the grid 181 is recorded with respect to a position and orientation of the anatomy array 123, which can be attached to the patient's body at the second position 105B, at some distance away from the area of interest, and next the registration grid 181 can be removed from the patient. As a result, the anatomy array 123 is positioned at the second position 105B away from the operating area and does not disrupt the surgeon during the operation, while still allows to track the position and orientation of the anatomy array 123 and therefore determine the corresponding position and orientation of the patient anatomy subject to the operation.

Once the anatomy of the patient is registered for the operation, the virtual images can be correctly collocated with the real world image, such as shown in Figs. 3A, 3B.

## Claims

1. A method for registering patient anatomical data (163) in a surgical navigation system, the method comprising the following steps:
- by means of a medical scanner (180), scanning (502) a volume comprising a patient anatomy of interest and a registration grid (181) placed at a first position (105A) over the surgical field, to obtain patient anatomical data (163), wherein the registration grid (181) comprises a plurality of fiducial markers (181B);
- by means of a fiducial marker tracker (125), capturing (504) the 3D positions and orientations of a pre-attached anatomy array (123) attached to the patient at a second position (105B) around the surgical field and of the registration grid (181) placed over the surgical field (105A), wherein the anatomy array (123) comprises a plurality of fiducial markers (123B); and
- registering (506) the patient anatomical data (163) with respect to the 3D position and orientation of the anatomy array (123) determined as a function of the relative positions and orientations of the registration grid (181) and the anatomy array (123).

2. The method according to claim 1, wherein the fiducial marker tracker (125) uses an optical, electromagnetic, or acoustic technology for capturing (504) the 3D positions and orientations of the registration grid (181) and the anatomy array (123).

3. The method according to any of previous claims, further comprising:
- using the fiducial marker tracker (125) for obtaining in real-time the 3D position and orientation of the anatomy array (123);
- generating, by a surgical navigation image generator (131), a surgical navigation image (142A) comprising the patient virtual anatomical data (163) that is positioned and oriented with respect to the 3D position and orientation of the anatomy array (123); and
- showing the surgical navigation image (142A) by means of a 3D display system (140) such that an augmented reality image (141A), collocated with the surgical field (105A), is visible to a viewer looking towards the surgical field (105A).

4. The method according to claim 3, further comprising:
- by means of the fiducial marker tracker (125), real-time tracking of: a surgeon's head (106), a 3D display (142), and surgical instruments (107) to provide current 3D position and orientation data; and
- adjusting the surgical navigation image (142A) with respect to the tracked current 3D position and orientation data.

5. The method according to claim 3 or 4, further comprising:
- by means of the tracker (125), real-time tracking of a robotic arm marker array (126); and
- generating the surgical navigation image (142A), further comprising the robotic arm virtual image (166), positioned and oriented with respect to the tracked 3D position and orientation of the robotic arm marker array (126).

6. The method according to any of previous claims, further comprising identifying the fiducial markers of the registration grid (181) in the volumetric data provided by the medical scanner using a convolutional neural network (CNN).
